Europäisches Patentamt

European Patent Office

Office européen des brevets

(19)

(11) Publication number: **0 052 008**
**A1**

(12) # EUROPEAN PATENT APPLICATION

(21) Application number: **81305321.2**

(22) Date of filing: **10.11.81**

(51) Int. Cl.³: **A 61 M 1/03**

(30) Priority: **12.11.80 SE 8007921**

(43) Date of publication of application: **19.05.82**
**Bulletin 82/20**

(84) Designated Contracting States: **AT BE CH DE FR GB IT LI LU NL**

(71) Applicant: **ALFA-LAVAL AB, Box 500, S-147 00 Tumba (SE)**

(72) Inventor: **Rudena, Alf, Varpholmsgrand 65, S-127 46 Skarholmen (SE)**

(74) Representative: **Simpson, Ronald Duncan Innes et al, A.A.Thornton & Co. Northumberland House 303-306 High Holborn, London WCIV 7LE (GB)**

(54) Method of conducting dialyzate fluid to and from a dialysis membrane cassette and apparatus for carrying out the method.

(57) Dialyzate fluid is supplied to membrane cassettes (6) at respective dialysis treatment stations from a central preparation tank (2) by pumping (3) the fluid to a storage tank (4) from which it flows under gravity to the membrane cassettes (6) through a pipeline (5). The used fluid withdrawn from the membrane cassettes (6) is conducted under vacuum through a pipeline (9) to a container (7) connected to a vacuum pump (8). The necessary pressure differential in the membrane cassettes is created by the vacuum either by (i) providing a throttling valve (5a) on the inlet side of each cassette (6) and connecting the outlet directly to the vacuum line (9) or by using a vacuum driven dosing apparatus (11) which supplies clean fluid in dosed quantities to the cassette and withdraws used fluid in larger dosed quantities from the cassette.

ACTORUM AG

- 1 -

Method of conducting dialyzate fluid to and from
a dialysis membrane cassette and apparatus for
carrying out the method.

The present invention relates to a method of
transporting and distributing dialyzate fluid at which
the dialyzate fluid is prepared in a central part of a
plant and transported to a number of treatment places,
and an arrangement for carrying through this method.

Persons who lack or have a reduced kidney
function must have their blood treated regularly and
have a certain amount of liquid removed from the blood.
This dialysis treatment is normally performed two or
three times a week and each treatment takes four to six
hours. The dialysis usually involves two steps making
use of a membrane cassette consisting of a large number
of capillary tubes made of semipermeable membrane. In
the first step diffusion of unwanted salts from the
blood to the dialyzate fluid occurs by making use of a
relatively low pressure difference between the blood
and the dialyzate fluid. In the second step the pressure
difference is increased and the required amount of
liquid of between one to two litres per treatment passes
through the membrane and mixes with the dialyzate fluid.
The first step usually takes about 5 times as long as
the second step.

The modern dialysis treatment started around 1960 in the U.S.A. In the beginning plants adapted for central preparation of the dialyzate fluid and storage of the fluid in large tanks were used. Later it became more common to treat the patients at home by means of so-called home monitors. The dialysis at home had a number of disadvantages, one of which was that the patients felt that they had lost their contact with the hospital. The most popular way of carrying out dialysis treatment is now in hospitals using plants with central preparation and storage of dialyzate fluid.

A medical demand is that even when dialysis plants with a central preparation and storage of the dialyzate fluid are used, it should be possible to offer a personalised treatment, with the result that units intended for home dialysis now are used at each treatment place in the hospitals. These units require water supply and drainage facilities at each treatment place, which makes the installation as a whole more expensive and more complex.

The cleaning of the plants in current use and having central preparation and storage of the dialyzate fluid is performed manually and causes a lot of problems. It is also time consuming and expensive, making it difficult for the cleaning to be properly carried out as often as is desirable.

The aim of the present invention is to eliminate or reduce the drawbacks of the known dialysis installations and the method according to the invention is characterised in that the dialyzate fluid is pumped from a preparation tank to a storage tank, the fluid flows under gravity from the storage tank and to a treatment station from where it is fed to the membrane cassette, used dialyzate fluid is drawn from the membrane cassette and is conducted by vacuum to a container, the container being connected to a vacuum source and having a discharge outlet, and the vacuum source is adapted to produce the necessary pressure difference in the membrane cassette between the blood, under treatment, and the dialyzate fluid.

The apparatus provided in accordance with the invention for carrying out the method is characterised by a storage tank, a first pipe line connecting the storage tank to the membrane cassette for the dialyzate fluid to flow therethrough under gravity and to pass to the membrane cassette, a second pipe line for conducting used dialyzate fluid withdrawn from the membrane cassette to a container, and a vacuum pump connected to the container to produce a vacuum in the second pipe line for transporting the fluid therethrough.

The advantages of the invention are that it:-
- can be easily installed in new and old premises without any expensive changes or rebuildings;
- may be of considerable advantage to the staff on the dialysis department as cleaning and supervision can be simpler;
- allows individual treatment;
- can increase the hygienic security by allowing for an automatic and efficient cleaning system;
- can increase the efficiency of the membrane cassette through an intermittent, pulsating manner of operation; and
- can improve the accuracy and control of the right concentration of dialyzate fluid.

During the treatment salts are transferred from the blood by diffusion and liquid by ultrafiltration to the dialyzate fluid. The used dialyzate fluid is transported by means of vacuum from the different treatment stations to a container which serves as an air separator and is connected to the vacuum

- 4 -

pump. From the tank the used dialyzate fluid is discharged to the drainage system of the building.

With this method of conducting the dialyzate fluid to the different treatment places by means of gravity and exhausting the used dialyzate fluid by suction, space occupying transporting arrangements on and under the floors may be avoided and the pipelines can be located instead above the membrane cassettes and the treatment stations. It is also possible to avoid expensive manual handling and the use of a pump at each treatment station. The installation with central preparation and storage of dialyzate fluid is very flexible and may be easily adapted to suit the location of the treatment stations in the premises and further treatment stations can be easily added at low costs.

The installation is also much simpler to clean in an efficient way, at the same time requiring smaller amounts of water and cleaner, which lowers the cleaning costs. An automatic cleaning device can be used in combination with the vacuum system in the plant to cause small amounts of cleaner and rinsing water to rush through the pipelines at a high rate and at repeated intervals, which is more efficient than filling the system with cleaner or rinsing water and pumping it around.

The dialyzate fluid can be prepared by dosing a dialyzate concentrate and water in right proportions in a dosing arrangement known for example through the Swedish patent No. 415,934.

The blood may with advantage be treated by letting it flow within the capillary tubes consisting of membranes in the membrane cassette, while dialyzate fluid surrounds the outside of the capillary tubes and is at a lower pressure than the blood. This pressure difference can be created by the vacuum in one of the two following ways:-

i) passing the dialyzate fluid through a throttling valve on the inlet side of the membrane cassette,

ii) passing the dialyzate fluid through a dosing arrangement as described in the Swedish patent mentioned above and which is driven by the vacuum system.

The dosing arrangement comprises two pistons, the first one transports the dialyzate fluid to the membrane cassette and the second piston sucks the used dialyzate fluid out from the membrane cassette. The pistons are coupled together mechanically and the ratio of their stroke lengths is infinitely variable. If the volume of fluid withdrawn from the membrane cassette during any stroke is greater than the volume of fluid supplied there is created an under pressure in the dialyzate fluid within the membrane cassette. The difference in volume of the piston strokes is the amount of liquid by which the volume of blood is diminished per stroke in the membrane cassette. It is possible to provide the dosing arrangement with a programmed control such that it operates at first with only a small difference in volume of the piston strokes and thereafter with a larger difference. In the first step diffusion with little removal of liquid from the blood is obtained, while an ultrafiltration with increased removal of liquid takes place in the second step. It is also possible to program the dosing arrangement such that the difference in volume of stroke is integrated over the time and is recorded so that the amount of liquid removed from the blood is easily determined. This creates possibilities for individual treatment and easier supervision of the patient. It is consequently always possible to supervise the amount of liquid removed from the blood. The dosing arrangement also makes it possible to recirculate the dialyzate fluid such that a dialyzate fluid which has been used at one treatment station may be mixed with clean dialyzate fluid to be reused at the same station. Recirculation of the dialyzate fluid reduces the consumption of dialyzate fluid and hence the cost.

The necessary pressure difference in the cassette membrane must not be created by putting the blood under relatively high pressure while the dialyzate fluid remains at atmospheric pressure. Should an error occur in the system on the blood side an over pressure may injure the patient. This risk does not occur with the described method.

With the method of the invention the dialyzate fluid can be made to work intermittently, i.e. pulsate through the membrane cassette. The pulsation may be achieved by means of a special pulsator in the vacuum system when the pressure difference is obtained by means of a throttling valve according to (i) above, while the dosing arrangement described above produces automatically a pulsating flow of liquid. A pulsating flow acts such that:-

- the dialyzate fluid is distributed in a better way among the capillary tubes in the membrane cassette;
- the concentration polarization around the capillary tube is counteracted;
- air bubbles which may enter with the dialyzate fluid and settled on and rendered inactive a part of the membrane surface are driven away.

All three factors increase the efficiency of the membrane cassette and make it possible to work with considerably smaller flows of dialyzate fluid. Hitherto flows of 1 to 2 l/min. have been used whereas flow rates of 100-200 ml/min. are possible.

A full understanding of the invention will be had from the following detailed description which is given with reference to the accompanying drawings, in which:-

Figure 1 shows schematically an installation embodying the invention; and

Figure 2 shows schematically an embodiment in which a dosing arrangement is used at each treatment station.

For reasons of simplicity obvious details as valves and the like have not been shown in the drawings.

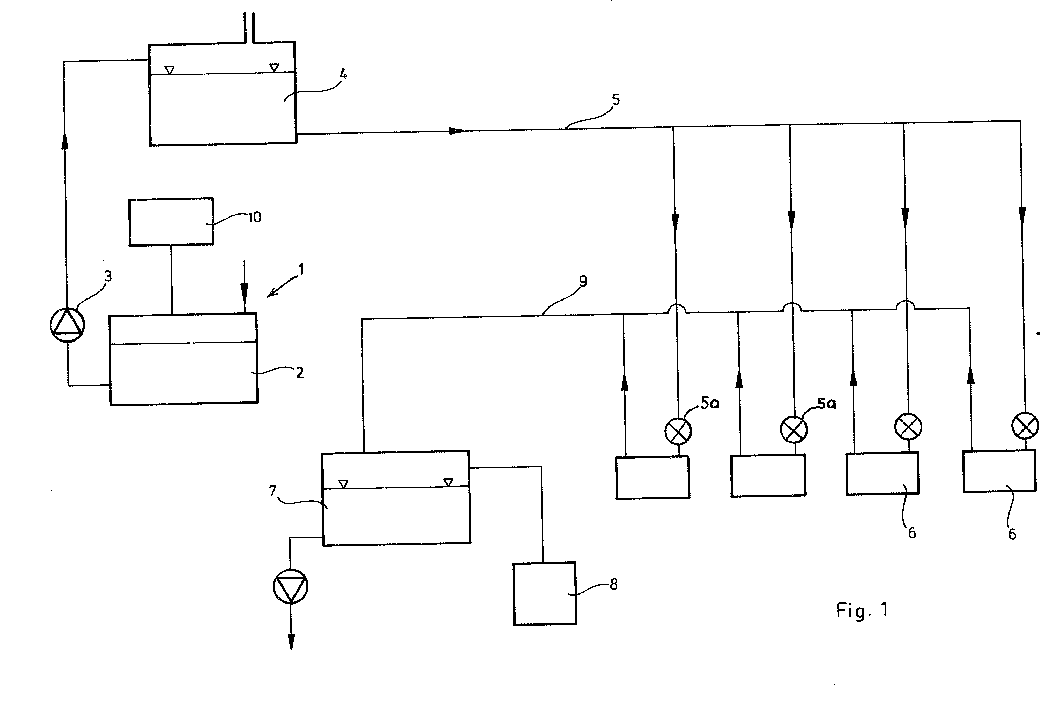

The installation illustrated in Figure 1 includes equipment 1 for central preparation of dialyzate fluid which includes a tank 2 and a pump 3 for transporting the fluid from tank 2 to a storage tank 4 having means to control the level of fluid therein. The dialyzate fluid flows from the tank 4 under gravity by way of a pipeline 5 to throttling valves 5a and membrane cassettes 6 located at respective treatment stations. The used dialyzate fluid is drawn by vacuum from the membrane cassettes 6 through a pipeline 9 to a container 7 connected to a vacuum pump 8. The container 7 is equipped with means to control the level of fluid therein and from the container the salt containing dialyzate fluid is transported and discharged to drain. An automatic cleaning device 10 is provided for cleaning of all the equipment which is connected to it, for example the preparation equipment 1, the preparation tank 2, the dialyzate pump 3, the storage tank 4, the membrane cassettes 6, the container 7 and all the pipelines which interconnect these parts of the installation.

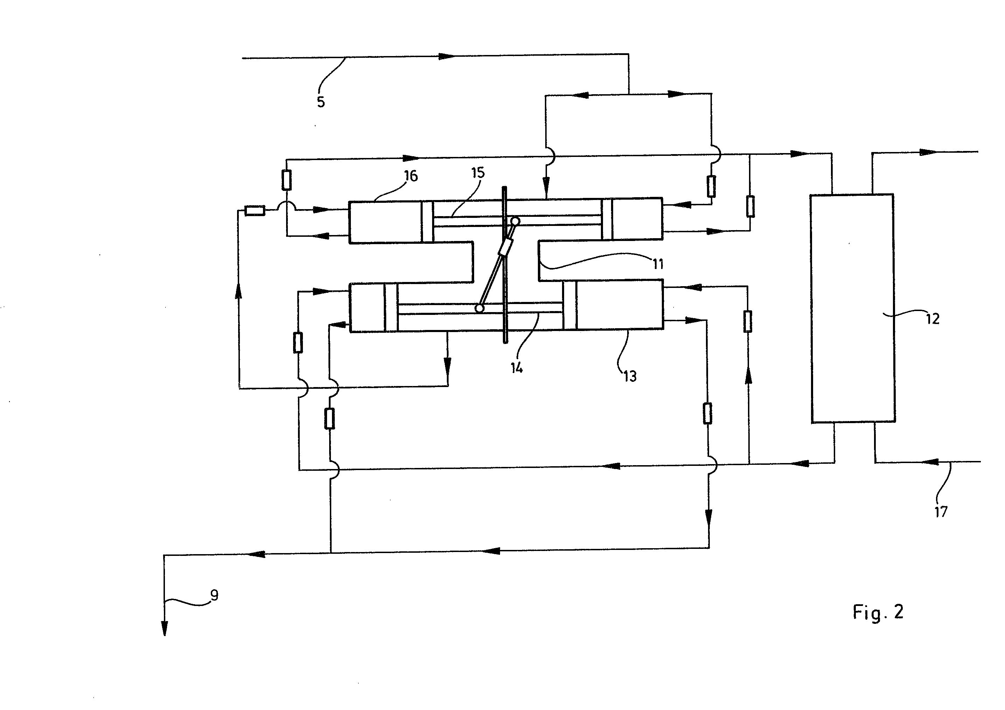

Figure 2 illustrates a dosing arrangement 11 which is arranged to be driven by the vacuum system by way of the pipeline 9, and works together with a membrane filter 12. The salt containing dialyzate fluid is drawn alternately from one and then the other end of a cylinder 13 into the pipeline 9 while used dialyzate fluid enters the opposite end of the cylinder 13 from the membrane filter. The piston 14 is moved backwards and forwards as used dialyzate fluid is withdrawn from the opposite ends of the cylinder 13. The piston 14 is coupled mechanically to a second piston 15 in a cylinder 16 so that movement of the piston 14 is transmitted to the piston 15. The mechanical coupling is so arranged that the two pistons may have the same or different stroke lengths. As piston 15 reciprocates, clean dialyzate fluid from the pipeline 5 enters the alternate ends of the cylinder 16 and is discharged from the opposite end of cylinder 16 to the membrane filter 12. In this way metered doses of clean dialyzate fluid are supplied to the membrane filter 12. Blood is led through a pipe to the other side of the membrane filter 12. The necessary valves and control of them are shown only diagrammatically in Figure 2.

Claims:

1.        A method of conducting dialyzate fluid to
and from a membrane cassette (6) in an installation in
which dialyzate fluid is prepared centrally and then
supplied to a number of treatment stations,
characterised in that the dialyzate fluid is pumped
from a preparation tank (2) to a storage tank (4),
the fluid flows under gravity from the storage tank (4)
and to a treatment station from where it is fed to the
membrane cassette (6), used dialyzate fluid is drawn
from the membrane cassette (6) and is conducted by
vacuum to a container (7), the container being connected
to a vacuum source (8) and having a discharge outlet,
and the vacuum source (8) is adapted to produce the
necessary pressure difference in the membrane cassette
between the blood, under treatment, and the dialyzate
fluid.

2.        A method according to claim 1, wherein the
necessary pressure difference in the membrane cassette
(6) is obtained by providing at the treatment station
a throttling valve (5a) in a pipeline connecting the
storage tank to the membrane cassette, and withdrawing
used dialyzate fluid from the membrane cassette under
the vacuum produced by the vacuum source (8).

3.        A method according to claim 1, wherein the
necessary pressure difference in the membrane cassette
is obtained by means of a vacuum driven dosing
apparatus (11) arranged to supply clean dialyzate fluid
to the membrane cassette in dosed quantities and to
withdraw used dialyzate fluid from the membrane
cassette in larger dosed quantities.

4.        A method according to claim 1 or 2, wherein the dialyzate fluid is conducted through the membrane cassette with a pulsating flow.

5.        An apparatus for carrying out the method of claim 1, comprising a central preparation tank (2) and a membrane cassette (6), and characterised by a storage tank (4), a first pipeline (5) connecting the storage tank to the membrane cassette (6) for the dialyzate fluid to flow therethrough under gravity and to pass to the membrane cassette, a second pipeline (9) for conducting used dialyzate fluid withdrawn from the membrane cassette to a container (7), and a vacuum pump (8) connected to the container (7) to produce a vacuum in the second pipeline (9) for transporting the fluid therethrough.

6.        An apparatus according to claim 5, wherein the pipelines (5,9) are located above the membrane cassette.

7.        An apparatus according to claim 5 or 6, wherein a dosing device (11) is connected to the membrane cassette (6) and includes one dosing cylinder for supplying dosed quantities of clean dialyzate fluid to the membrane cassette, another cylinder for withdrawing used dialyzate fluid in dosed quantities from the membrane cassette, and means to control the relative volumes of the dosed quantities supplied and withdrawn by the cylinders.

8.        An apparatus according to claim 7, wherein the control means is arranged to integrate the difference in dosed volumes and record the result to indicate the amount of liquid removed from the blood being treated.

9.        An apparatus according to claim 5 or 6, wherein a throttling valve (5a) is connected in the first pipeline at the inlet to the membrane cassette (6) and the outlet of the membrane cassette is connected directly to the second pipeline (9).

10.       An apparatus according to any one of claims 5 to 8, wherein an automatic cleaning device is provided for cleaning at least some of the parts thereof.

Fig. 1

1/2

0052008

Fig. 2

0052008

| Category | Citation of document with indication, where appropriate, of relevant passages | Relevant to claim |
|---|---|---|
| Y | US - A - 3 441 136 (MILTON) | 1 |
| A | * see figure 2; claim 1, lines 39-45; column 6, lines 25,26,29,30,51,52,67,68, 74 and 75; column 7, lines 1,2, 48,49,68 and 69; | 2,5,9 |
| A | see abstract * | 10 |
| Y | US - A - 3 605 783 (BIO/SYSTEMS) | |
| | * see figure 1; column 2, lines 41-45 * | 1 |
| Y | GB - A - 2 034 601 (CORDIS DOW) | |
| | * see page 4, line 63; | 1 |
| A | see page 3, lines 22-38; see page 4, lines 58-64 * | 3,7 |

**DOCUMENTS CONSIDERED TO BE RELEVANT**

**CLASSIFICATION OF THE APPLICATION (Int. Cl. 3)**

A 61 M 1/03

**TECHNICAL FIELDS SEARCHED (Int.Cl. 3)**

A 61 M

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

The present search report has been drawn up for all claims

| Place of search | Date of completion of the search | Examiner |
|---|---|---|
| The Hague | 02.02.1982 | PETZUCH |

EPO Form 1503.1  06.78